# NOUVEAU FASCICULE DE BREVET EUROPEEN

(11) **EP 0 763 116 B2**
(45) Date de publication et mention de la décision concernant l'opposition: **23.04.2008**
(45) Mention de la délivrance du brevet: 03.09.2003
(21) Numéro de dépôt: 95920972.7
(22) Date de dépôt: 22.05.1995
(51) Int. Cl.: C12N 15/55, A61K 38/43, C12N 15/86, C12N 5/10, A61K 48/00, A61K 9/22, C12N 7/01, C12R 1/91

(54) **VIRUS RECOMBINANTS, PREPARATION ET UTILISATION EN THERAPIE GENIQUE**
REKOMBINANTE VIREN, IHRE HERSTELLUNG UND IHRE VERWENDUNG IN DER GENTHERAPIE
RECOMBINANT VIRUSES, PREPARATION AND USE THEREOF IN GENE THERAPY

(30) Priorité: 02.06.1994 FR 9406759
(43) Date de publication de la demande: 19.03.1997
(73) Titulaire: Aventis Pharma S.A., 92160 Antony (FR); THE UNIVERSITY OF BRITISH COLUMBIA, Vancouver, British Columbia V6T 1Z1 (CA)
(72) Inventeur: BENOIT, Patrick, F-75014 Paris (FR); DENEFLE, Patrice, F-94100 Saint-Maur (FR); PERRICAUDET, Michel, F-28320 Ecrosnes (FR); HAYDEN, Michael R., Vancouver BC, V6R 1W9 (CA); LEWIS, Marion Elizabeth Suzanne, Vancouver BC, V7W 2N2 (CA)
(74) Mandataire: Bouvet, Philippe
(86) Numéro de dépôt international: PCT/FR1995/000669
(87) Numéro de publication internationale: WO 1995/033840

(56) Documents cités:
- WO-A-93/00426
- JOURNAL OF BIOLOGICAL CHEMISTRY, vol.265, no.10, 1990, BALTIMORE, MD US pages 5429 - 5433 SEMENKOVICH C. F. ET AL. 'In Vitro expression and site-specific mutagenesis of the cloned human lipoprotein lipase gene'
- JOURNAL OF BIOLOGICAL CHEMISTRY, vol.268, no.5, 15 Février 1993, BALTIMORE, MD US pages 3272 - 3276 BERRYMAN D. E. ET AL. 'Site-directed mutagenesis of a putative heparin binding domain of avian lipoprotein lipase'
- CLINICAL RESEARCH, vol.41, no.2, Avril 1993 page 206A MAEDA H. ET AL. 'Gene therapy strategy for the pancreatic insufficiency of cystic fibrosis: Adenovirus-mediated transfer of the human pancreatic lipase cDNA'
- JOURNAL OF BIOLOGICAL CHEMISTRY, vol.264, no.33, 1989, BALTIMORE, MD US pages 20042 - 20048 LOWE M. E. ET AL. 'Cloning and characterization of human pancreatic lipase cDNA'
- PROCEEDINGS OF THE NATIONAL ACADEMY OF SCIENCES OF USA, vol.87, 1990, WASHINGTON US pages 3474 - 3478 BEG O. U. ET AL. 'Lipoprotein lipase Bethesda: a single amino acid substitution (Ala-176-Thr) leads to abnormal heparin binding and loss of enzymic activity'
- PROTEIN ENGINEERING, vol.7, Avril 1994, ENGLAND GB pages 537 - 541 HOLM C. ET AL. 'Hormone-sensitive lipase: structure, function, evolution and overproduction in incect cells using the baculovirus expression system'
- JOURNAL OF BIOLOGICAL CHEMISTRY, vol.267, no.2, 1992, BALTIMORE, MD US pages 963 - 967 WILSON J. M. ET AL. 'Hepatocyte-directed gene transfer in Vivo leads to transient improvement of hypercholesterolimia in low density lipoprotein receptor-deficient rabbits'
- PEDIATRIC RESEARCH, vol.34, no.4, 1993 pages 403 - 415 HUMPHRIES S. E. ET AL. 'The molecular genetics of pediatric lipid disorders: recent progress and future research directions'
- HUMAN GENE TRANSFER, vol.219, 1991 pages 51 - 61 STRATFORD-PERRICAUDET L. ET AL. 'Gene transfer into animals: the promise of adenovirus'

## Description

La présente invention concerne des vecteurs recombinants d'origine virale, leur préparation et leur utilisation, notamment pour le traitement et/ou la prévention des pathologies liées aux dyslipoprotéinémies. Plus particulièrement, elle concerne des virus recombinants comportant une séquence d'ADN codant pour une lipase impliquée dans le métabolisme des lipoprotéines. L'invention concerne également la préparation de ces vecteurs, les compositions pharmaceutiques les contenant et leur utilisation thérapeutique, notamment en thérapie génique.

Les dyslipoprotéinémies sont des désordres du métabolisme des lipoprotéines responsables du transport dans le sang et les fluides périphériques de lipides comme le cholestérol et les triglycérides. Elles conduisent à des pathologies importantes, liées respectivement à l'hypercholestérolémie ou l'hypertriglycéridémie, telles que notamment l'athérosclérose. L'athérosclérose est une maladie complexe, polygénique, qui est définie sur le plan histologique par des dépôts (plaques lipidiques ou fibro-lipidiques) de lipides et d'autres dérivés sanguins dans la paroi des grosses artères (aorte, artères coronaires, carotide). Ces plaques, plus ou moins calcifiées selon l'avancement du processus, peuvent être jumelées à des lésions et sont liées à l'accumulation dans les artères de dépots graisseux constitués essentiellement d'esters de cholestérol. Ces plaques s'accompagnent d'un épaississement de la paroi artérielle, avec hypertrophie du muscle lisse, apparition de cellules spumeuses et accumulation de tissu fibreux. La plaque athéromateuse est très nettement en relief sur la paroi, ce qui lui confère un caractère sténosant responsable des occlusions vasculaires par athérome, thrombose ou embolie qui surviennent chez les patients les plus atteints. Les dyslipoprotéinémies peuvent donc conduire à des pathologies cardio-vasculaires très graves telles que l'infarctus, la mort subite, la décompensation cardiaque, les accidents cérébro-vasculaires, etc.

Actuellement, ces pathologies, et en particulier les hypercholestérolémies, sont traitées essentiellement au moyen de composés agissant soit sur la biosynthèse du cholestérol (inhibiteurs de l'hydroxyméthylglutaryl-coenzymeA reductase, les statines), soit sur la captation et l'élimination du cholestérol biliaire (les séquestrants ou résines), soit encore sur la lipolyse par un mode d'action qui reste à élucider sur le plan moléculaire (les fibrates). Par conséquent, toutes les grandes classes de médicaments, qui ont été utilisées dans cette indication (séquestrants, fibrates ou statines), s'adressent seulement à l'aspect préventif de la formation de la plaque d'athérome et non en fait au traitement de l'athérome. Les traitements actuels de l'athérome, post accident coronarien, ne sont que palliatifs puisqu'ils n'interviennent pas sur l'homéostase du cholestérol et qu'ils sont des actes chirurgicaux (by-pass coronarien, angioplastie).

La présente invention constitue une nouvelle approche thérapeutique pour le traitement des pathologies liées aux dyslipoprotéinémies. Elle propose une solution avantageuse aux inconvénients de l'art antérieur, en démontrant la possibilité de traiter les pathologies liées aux dyslipoprotéinémies par la thérapie génique, par le transfert et l'expression in vivo d'un gène codant pour une lipase impliquée dans le métabolisme des lipoprotéines. L'invention offre ainsi un moyen simple permettant un traitement spécifique et efficace de ces pathologies.

La thérapie génique consiste à corriger une déficience ou une anomalie (mutation, expression aberrante, etc) ou à assurer l'expression d'une protéine d'intérêt thérapeutique par introduction d'une information génétique dans la cellule ou l'organe affecté. Cette information génétique peut être introduite soit ex vivo dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J. Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

La présente invention constitue une nouvelle approche thérapeutique pour le traitement des pathologies liées aux dyslipoprotéinémies, consistant à transférer et à exprimer in vivo des gènes codant pour des lipases impliquées dans le métabolisme des lipoprotéines. De manière particulièrement avantageuse, la demanderesse a maintenant montré qu'il est possible de construire des virus recombinants contenant une séquence d'ADN codant pour une lipase impliquée dans le métabolisme des lipoprotéines, d'administrer ces virus recombinants in vivo, et que cette administration permet une expression stable et efficace d'une lipase biologiquement active in vivo, et sans effet cytopathologique.

La présente invention résulte également de la mise en évidence que les adénovirus constituent des vecteurs particulièrement efficaces pour le transfert et l'expression de tels gènes. En particulier, la présente invention montre que l'utilisation d'adénovirus recombinants comme vecteurs permet d'obtenir des niveaux d'expression suffisamment élevés de ces gènes pour produire l'effet thérapeutiqe recherché.

La présente invention offre ainsi une nouvelle approche pour le traitement et la prévention des pathologies cardiovasculaires et neurologiques liées aux dyslipoprotéinémies.

Un premier objet de l'invention réside donc dans un virus recombinant défectif comprenant une séquence nucléique codant pour une lipoprotéine lipase (LPL) impliquée dans le métabolisme des lipoprotéines.

L'invention a également pour objet l'utilisation d'un tel virus recombinant défectif pour la préparation d'une composition pharmaceutique destinée au traitement et/ou à la prévention des maladies cardiovasculaires.

La présente invention concerne également l'utilisation de cellules modifiées génétiquement ex vivo par un virus tel que décrit ci-dessus, ou de cellules productrices de tels virus, implantatées dans l'organisme, permettant une libération in vivo prolongée et efficace d'une lipase biologiquement active.

La lipoprotéine lipase (LPL) est une enzyme qui permet l'hydrolyse des triglycérides contenus dans les lipoprotéines de très basse densité (VLDL) ou les chylomicrons. L'apolipoprotéine CII, qui est présente à la surface de ces particules, est utilisée comme cofacteur dans cette hydrolyse. Naturellement, la LPL est principalement synthétisée par les adipocytes sous la forme d'un précurseur monomérique de 51 kDa, qui est ensuite glycosylé (58kDa). Dans le sang, la LPL est active sous forme dimérique. Jusqu'à 80% de la LPL fraîchement synthétisée est dégradée dans le compartiment lysosomal avant d'avoir pu être sécrétée. Après sa sécrétion, la LPL est captée par la face luminale des cellules de l'endothélium vasculaire où elle se fixe par l'intermédiaire de glycosaminoglycans. Elle a une très forte affinité pour l'héparine qui permet de déplacer la LPL de son site de fixation à la surface de la cellule endothéliale. L'injection intraveineuse d'héparine permet de mesurer la concentration et l'activité en LPL chez des patients. La LPL est utilisée au niveau des cellules vasculaires, mais aussi hépatiques comme capteur de lipoprotéines, augmentant leur rétention à la surface cellulaire et favorisant ainsi leur captation ou leur modification.

L'ADNc codant pour la LPL humaine a été cloné et séquencé (Wion et al, Science 235 (1987) 1638-1641). Deux formes de messagers de 3350 et 3750 bases coexistent, principalement dans le tissu adipeux et musculaire, et sont issus de l'utilisation de 2 sites de polyadénylation. Ils incluent une longue séquence 3' non-traduite et codent pour une préprotéine de 475 aa d'où est clivée une séquence leader de 27 aa pour donner naissance à la protéine monomérique mature de 448 résidus. Le gène de la LPL a également été cloné (Kirchgessner et al, Proc.Natl.Acad.Sci.USA, 1987, 262:9647-9651). La synthèse, le processing et la sécrétion de la LPL sont régulés de façon complexe pendant le développement et en réponse à des stimuli hormonaux. Une part importante de cette régulation est accomplie au niveau transcriptionnel. (revu dans Auwerx et al, Critical Reviews in Clinical Laboratory Sciences, 1992, 29:243-268)

La présente invention montre qu'il est possible d'incorporer une séquence d'ADN codant pour la LPL dans un vecteur viral, et que ces vecteurs permettent d'exprimer et de sécréter efficacement une forme mature, biologiquement active (dimérique) de la LPL. Plus particulièrement, l'invention montre que l'expression in vivo de LPL active peut être obtenue par administration directe d'un adénovirus ou par implantation d'une cellule productrice ou génétiquement modifiée par un adénovirus ou par un rétrovirus incorporant un tel ADN.

Les vecteurs de l'invention peuvent notamment être utilisés pour corriger des déficiences en LPL dues à mutations dans le gène de la LPL. De telles déficiences sont relativement fréquentes et peuvent atteindre une prévalence de 1:5000-1:10000 dans certaines populations (S. Santamarina-Fojo, 1992, Cur. Op. lipid., 3:186-195). Ces déficiences peuvent résulter d'une mutation importante au niveau du gène, conduisant à l'absence de synthèse de la LPL ou à la synthèse d'une protéine tronquée ou très modifiée. Il a en effet été montré l'existence chez certains malades de mutations de type insertions, délétions ou mutations non-sens (revue dans S. Santamarina-Fojo, 1992, Cur. Op. lipid., 3:186-195). Elles peuvent résulter également d'un défaut au niveau du site catabolique, pouvant être dû à des mutations de type "missense" au niveau du gène. Elles peuvent également résulter de modification tant au niveau du site de liaison à l'héparine qu'au niveau du site catalytique. Au stade hétérozygote, ces déficiences peuvent représenter une part importante des hyperlipidémies les plus communes dont les hypertriglycérinémies familiales, les hyperlipidémies familiales combinées et les hyperlipidémies postprandiales.

La présente invention est particulièrement avantageuse car elle permet d'induire une expression contrôlée et sans effet nocif de LPL dans des organes qui ne sont pas normalement concernés par l'expression de cette protéine. En particulier, une libération tout à fait avantageuse est obtenue par implantation de cellules productrices de vecteurs de l'invention, ou infectées ex vivo par des vecteurs de l'invention.

L'activité lipase produite dans le cadre de la présente invention peut être une lipase humaine ou animale. La séquence nucléique utilisée dans le cadre de la présente invention peut être un ADNc, un ADN génomique (ADNg), un ARN (dans le cas des rétrovirus) ou une construction hybride consistant par exemple en un ADNc dans lequel seraient insérés un ou plusieurs introns. Il peut également s'agir de séquences synthétiques ou semisynthétiques. De manière particulièrement avantageuse, on utilise un ADNc ou un ADNg. En particulier, l'utilisation d'un ADNg permet une meilleure expression dans les cellules humaines. Pour permettre leur incorporation dans un vecteur viral selon l'invention, ces séquences sont avantageusement modifiées, par exemple par mutagénèse dirigée, en particulier pour l'insertion de sites de restriction appropriés. Les séquences décrites dans l'art antérieur ne sont en effet pas construites pour une utilisation selon l'invention, et des adaptations préalables peuvent s'avérer nécessaires, pour obtenir des expressions importantes. Dans le cadre de la présente invention, on préfère utiliser une séquence nucléique codant pour une lipase humaine. Par ailleurs, il est également possible d'utiliser une construction codant pour un dérivé de ces lipases, en particulier un dérivé des LPL et LH humaines. La LH (lipase hépatique) est localisée à la surface des cellules endothéliales hépatiques. Elle se distingue de la LPL par son insensibilité à l'action activatrice de l'apoC-II. La LH est impliquée dans l'hydrolyse des lipides des IDL, ainsi que des lipides des HDL2 entraînant leur conversion en HDL3. Un dérivé de ces lipases comprend par exemple toute séquence obtenue par mutation, délétion et/ou addition par rapport à la séquence native, et codant pour un produit conservant l'activité lipasique. Ces modifications peuvent être réalisées par les techniques connues de l'homme du métier (voir techniques générales de biologie moléculaire ci-après). L'activité biologique des dérivés ainsi obtenus peut ensuite être aisément déterminée, comme indiqué notamment dans l'exemple 3. Les dérivés au sens de l'invention peuvent également être obtenus par hybridation à partir de banques d'acides nucléiques, en utilisant comme sonde la séquence native ou un fragment de celle-ci.

Ces dérivés sont notamment des molécules ayant une plus grande affinité pour leurs sites de fixation, des molécules présentant une plus grande résistance aux protéases, des molécules ayant une efficacité thérapeutique plus grande ou des effets secondaires moindres, ou éventuellement de nouvelles propriétés biologiques. Les dérivés incluent également les séquences d'ADN modifiées permettant une expression améliorée in vivo.

Parmi les dérivés préférés, on peut citer plus particulièrement les variants naturels, les molécules dans lesquelles certains sites de N- ou O-glycosylation ont été modifiés ou supprimés, les molécules dans lesquelles un ou plusieurs résidus ont été substitués, ou les molécules dans lesquelles tous les résidus cystéine ont été substitués (mutéines). On peut citer également les dérivés obtenus par délétion de régions n'intervenant pas ou peu dans l'interaction avec les sites de liaison considérés ou exprimant une activité indésirable, et les dérivés comportant par rapport à la séquence native des résidus supplémentaires, tels que par exemple un signal de sécrétion et/ou un peptide de jonction.

Dans un premier mode de réalisation, la présente invention concerne un virus recombinant défectif comprenant une séquence d'ADNc codant pour une lipase impliquée dans le métabolisme des lipoprotéines. Dans un autre mode de réalisation préféré de l'invention, la séquence d'ADN est une séquence d'ADNg.

Les vecteurs de l'invention peuvent être préparés à partir de différents types de virus. Préférentiellement, on utilise des vecteurs dérivés des adénovirus, des virus adéno-associés (AAV), des virus de l'herpès (HSV) ou des rétrovirus. Il est tout particulièrement avantageux d'utiliser un adénovirus, pour une administration directe ou pour la modification ex vivo de cellules destinées à être implantées, ou un rétrovirus, pour l'implantation de cellules productrices.

Les virus selon l'invention sont défectifs, c'est-à-dire qu'ils sont incapables de se répliquer de façon autonome dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence nucléique codant pour la lipase. Préférentiellement, le virus défectif conserve néanmoins les séquences de son génome qui sont nécessaires à l'encapsidation des particules virales.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte. Préférentiellement, les adénovirus défectifs de l'invention comprenent les ITR, une séquence permettant l'encapsidation et la séquence codant pour la lipase. Avantageusement, dans le génome des adénovirus de l'invention, la région E1 au moins est rendue non fonctionnelle. Encore plus préférentiellement, dans le génome des adénovirus de l'invention, le gène E1 et au moins l'un des gènes E2, E4, L1-L5 sont non fonctionnels. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697) et L5 (WO95/02697). Selon un mode préféré de mise en oeuvre, l'adénovirus selon l'invention comprend une délétion dans les régions E1 et E4 et la séquence codant pour la LPL est insérée au niveau de la région E1 inactivée. Selon un autre mode de réalisation préféré, il comprend une délétion dans région E1 au niveau de laquelle sont insérés la région E4 et la séquence codant pour la LPL (Cf FR94 13355).

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN codant pour la lipase. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697.

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Toutefois, aucun de ces documents ne décrit ni ne suggère l'utilisation d'un AAV recombinant pour le transfert et l'expression in vivo ou ex vivo d'une lipase, ni les avantages d'un tel transfert. Les AAV recombinants défectifs selon l'invention peuvent être préparés par co-transfection, dans un lignée cellulaire infectée par un virus auxiliaire humain (par exemple un adénovirus), d'un plasmide contenant la séquence codant pour la lipase bordée de deux régions répétées inversées (ITR) d'AAV, et d'un plasmide portant les gènes d'encapsidation (gènes rep et cap) d'AAV. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc.

En particulier, les rétrovirus sont des virus intégratifs, infectant les cellules en division. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants comportant une séquence codant pour la LPL selon l'invention, un plasmide comportant notamment les LTR, la séquence d'encapsidation et ladite séquence codante est généralement construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

Pour la mise en oeuvre de la présente invention, il est tout particulièrement avantageux d'utiliser un adénovirus recombinant défectif. Les résultats donnés ci-après démontrent en effet les propriétés particulièrement intéressantes des adénovirus pour l'expression in vivo d'une protéine ayant une activité lipase. Les vecteurs adénoviraux selon l'invention sont particulièrement avantageux pour une administration directe in vivo d'une suspension purifiée, ou pour la transformation ex vivo de cellules, notamment autologues, en vue de leur implantation. De plus, les vecteurs adénoviraux selon l'invention présentent en outre des avantages importants, tels que notamment leur très haute efficacité d'infection, permettant de réaliser des infections à partir de faibles volumes de suspension virale. Dans un mode de réalisation particulièrement préféré, on utilise selon l'invention un adénovirus comportant en plus du gène codant pour la lipase un gène codant pour une apolipoprotéine. Il s'agit préférentiellement de la lipase hépatique et d'une apolipoprotéine sélectionnée parmi l'apoA-I et l'apoAIV. Les deux gènses sont avantageusement utilisés sous forme d'une construction bicistronique qui est introduite dans un vecteur adénoviral selon le protocole décrit ci-avant pour la construction d'un adénovirus comportant un seul gène. Avantageusement, l'invention concerne un adénovirus recombinant comportant un gène codant pour la LH et un gène codant pour l'ApoA-I insérés dans la région E1. La construction d'adénovirus comportant un gène codant pour une apo a été décrite dans la demande PCT/FR94/00422 qui est incorporée à la présente par référence.

Selon un autre mode particulièrement avantageux de mise en oeuvre de l'invention, on utilise une lignée productrice de vecteurs rétroviraux contenant la séquence codant pour la lipase, pour une implantation in vivo. Les lignées utilisables à cet effet sont notamment les cellules PA317 (US4,861,719), PsiCrip (WO90/02806) et GP+envAm-12 (US5,278,056), modifiées pour permettre la production d'un rétrovirus contenant une séquence nucléique codant pour une lipase selon l'invention.

Avantageusement, dans les vecteurs de l'invention, la séquence codant pour la lipase est placée sous le contrôle de signaux permettant son expression dans les cellules infectées. Il peut s'agir de signaux d'expression homologues ou hétérologues, c'est-à-dire de signaux différents de ceux naturellement responsables de l'expression de la lipase. Il peut s'agir en particulier de séquences responsables de l'expression d'autres protéines, ou de séquences synthétiques. Notamment, il peut s'agir de séquences de gènes eucaryotes ou viraux ou de séquences dérivées, stimulant ou réprimant la transcription d'un gène de façon spécifique ou non et de façon inductible ou non. A titre d'exemple, il peut s'agir de séquences promotrices issues du génome de la cellule que l'on désire infecter, ou du génome d'un virus, et notamment, les promoteurs des gènes E1A, MLP d'adénovirus, le promoteur CMV, LTR-RSV, etc. Parmi les promoteurs eucaryotes, on peut citer également les promoteurs ubiquitaires (HPRT, vimentine, α-actine, tubuline, etc), les promoteurs des filaments intermédiaires (desmine, neurofilaments, kératine, GFAP, etc) les promoteurs de gènes thérapeutiques (type MDR, CFTR, facteur VIII, etc) les promoteurs spécifiques de tissus (pyruvate kinase, villine, promoteur de la protéine intestinale de liaison des acides gras, promoteur de l'actine α des cellules du muscle lisse, promoteurs spécifiques pour le foie ; Apo AI, Apo AII, Albumine humaine etc) ou encore les promoteurs répondant à un stimulus (récepteur des hormones stéroïdes, récepteur de l'acide rétinoïque, etc,). En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Dans un mode particulier de réalisation, l'invention concerne un virus recombinant défectif comprenant une séquence nucléique codant pour une lipase impliquée dans le métabolisme des lipoprotéines sous le contrôle d'un promoteur choisi parmi le LTR-RSV ou le promoteur précoce du CMV.

Plus préférentiellement, la séquence nucléique utilisée comprend également des signaux permettant la sécrétion de la lipase par les cellules infectées. A cet effet, la séquence nucléique comporte généralement, en amont de la séquence codante, une séquence signal dirigeant la lipase synthétisée dans les voies de sécrétion de la cellule infectée. Cette séquence signal peut être la séquence signal naturelle de la lipase synthétisée, mais il peut également s'agir de toute autre séquence signal fonctionnelle dans la cellule infectée, ou d'une séquence signal artificielle.

Comme indiqué ci-avant, la présente invention concerne également toute utilisation d'un virus tel que décrit ci-dessus pour la préparation d'une composition pharmaceutique destinée au traitement et/ou à la prévention des pathologies liées aux dyslipoprotéinémies.

La présente invention concerne également une composition pharmaceutique comprenant un ou plusieurs virus recombinants défectifs tels que décrits précédemment. Ces compositions pharmaceutiques peuvent être formulées en vue d'administrations par voie topique, orale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection intraveineuse, telle que par exemple dans la veine porte du patient. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. L'injection directe dans la veine porte du patient est avantageuse car elle permet de cibler l'infection au niveau du foie et ainsi, de concentrer l'effet thérapeutique au niveau de cet organe.

Les doses de virus recombinant défectif utilisées pour l'injection peuvent être adaptées en fonction de différents paramètres, et notamment en fonction du vecteur viral, du mode d'administration utilisé, de la pathologie concernée ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

Concernant les rétrovirus, les compositions selon l'invention peuvent comporter directement les cellules productrices, en vue de leur implantation.

A cet égard, un autre objet de l'invention concerne toute cellule de mammifère infectée par un ou plusieurs virus recombinants défectifs tels que décrits ci-dessus. Plus particulièrement, l'invention concerne toute population de cellules humaines infectée par ces virus. Il peut s'agir en particulier de fibroblastes, myoblastes, hépatocytes, kératinocytes, cellules endothéliales, cellules gliales, etc.

Les cellules selon l'invention peuvent être issues de cultures primaires. Celles-ci peuvent être prélevées par toute technique connue de l'homme du métier, puis mises en culture dans des conditions permettant leur prolifération. S'agissant plus particulièrement de fibroblastes, ceux-ci peuvent être aisément obtenus à partir de biopsies, par exemple selon la technique décrite par Ham [Methods Cell.Biol. 21a (1980) 255]. Ces cellules peuvent être utilisées directement pour l'infection par les virus, ou conservées, par exemple par congélation, pour l'établissement de banques autologues, en vue d'une utilisation ultérieure. Les cellules selon l'invention peuvent également être des cultures secondaires, obtenues par exemple à partir de banques préétablies (voir par exemple EP 228458, EP 289034, EP 400047, EP 456640).

Les cellules en culture sont ensuite infectées par les virus recombinants, pour leur conférer la capacité de produire une lipase biologiquement active. L'infection est réalisée in vitro selon des techniques connues de l'homme du métier. En particulier, selon le type de cellules utilisé et le nombre de copies de virus par cellule désiré, l'homme du métier peut adapter la multiplicité d'infection et éventuellement le nombre de cycles d'infection réalisé. Il est bien entendu que ces étapes doivent être effectuées dans des conditions de stérilité appropriées lorsque les cellules sont destinées à une administration in vivo. Les doses de virus recombinant utilisées pour l'infection des cellules peuvent être adaptées par l'homme du métier selon le but recherché. Les conditions décrites ci-avant pour l'administration in vivo peuvent être appliquées à l'infection in vitro. Pour l'infection par des rétrovirus, il est également possible de co-cultiver les cellules que l'on désire infecter avec des cellules productrices des rétrovirus recombinants selon l'invention. Ceci permet de s'affranchir de la purification des rétrovirus.

Un autre objet de l'invention concerne un implant comprenant des cellules de mammifères infectées par un ou plusieurs virus recombinants défectifs telles que décrites ci-dessus ou des cellules productrices de virus recombinants, et une matrice extracellulaire. Préférentiellement, les implants selon l'invention comprennent 10⁵ à 10¹⁰ cellules. Plus préférentiellement, ils en comprennent 10⁶ à 10⁸.

Plus particulièrement, dans les implants de l'invention, la matrice extracellulaire comprend un composé gélifiant et éventuellement un support permettant l'ancrage des cellules.

Pour la préparation des implants selon l'invention, différents types de gélifiants peuvent être employés. Les gélifiants sont utilisés pour l'inclusion des cellules dans une matrice ayant la constitution d'un gel, et pour favoriser l'ancrage des cellules sur le support, le cas échéant. Différents agents d'adhésion cellulaire peuvent donc être utilisés comme gélifiants, tels que notamment le collagène, la gélatine, les glycosaminoglycans, la fibronectine, les lectines, etc. De préférence, on utilise dans le cadre de la présente invention du collagène. Il peut s'agir de collagène d'origine humaine, bovine ou murine. Plus préférenciellement, on utilise du collagène de type I.

Comme indiqué ci-avant, les compositions selon l'invention comprennent avantageusement un support permettant l'ancrage des cellules. Le terme ancrage désigne toute forme d'interaction biologique et/ou chimique et/ou physique entraînant l'adhésion et/ou la fixation des cellules sur le support. Par ailleurs, les cellules peuvent soit recouvrir le support utilisé, soit pénétrer à l'intérieur de ce support, soit les deux. On préfère utiliser dans le cadre de l'invention un support solide, non toxique et/ou bio-compatible. En particulier, on peut utiliser des fibres de polytétrafluoroéthylène (PTFE) ou un support d'origine biologique.

Les implants selon l'invention peuvent être implantés en différents sites de l'organisme. En particulier, l'implantation peut être effectuée au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse. Les implants selon l'invention sont particulièrement avantageux en ce sens qu'ils permettent de contrôler la libération de la lipase dans l'organisme : Celle-ci est tout d'abord déterminée par la multiplicité d'infection et par le nombre de cellules implantées. Ensuite, la libération peut être contrôlée soit par le retrait de l'implant, ce qui arrête définitivement le traitement, soit par l'utilisation de systèmes d'expression régulables, permettant d'induire ou de réprimer l'expression des gènes thérapeutiques.

La présente invention offre ainsi un moyen très efficace pour le traitement ou la prévention des pathologies liées aux dyslipoprotéinémies, en particulier l'obésité, l'hypertriglycéridémie, ou, dans le domaine des affections cardiovasculaires, l'infarctus du myocarde, l'angor, la mort subite, la décompensation cardiaque et les accidents cérébro-vasculaires.

En outre, ce traitement peut concerner aussi bien l'homme que tout animal tel que les ovins, les bovins, les animaux domestiques (chiens, chats, etc), les chevaux, les poissons, etc.

La présente invention sera plus complètement décrite à l'aide des exemples qui suivent, qui doivent être considérés comme illustratifs et non limitatifs.

### Légende des figures

Figure 1 : Structure du vecteur pXL2418
Figure 2 : Structure du vecteur pXL2419
Figure 3 : Structure du vecteur pXL CMV-LPL
Figure 4 : Structure du vecteur pXL RSV-LPL
Figure 5 : Structure du vecteur pXL RSV-LPLc

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

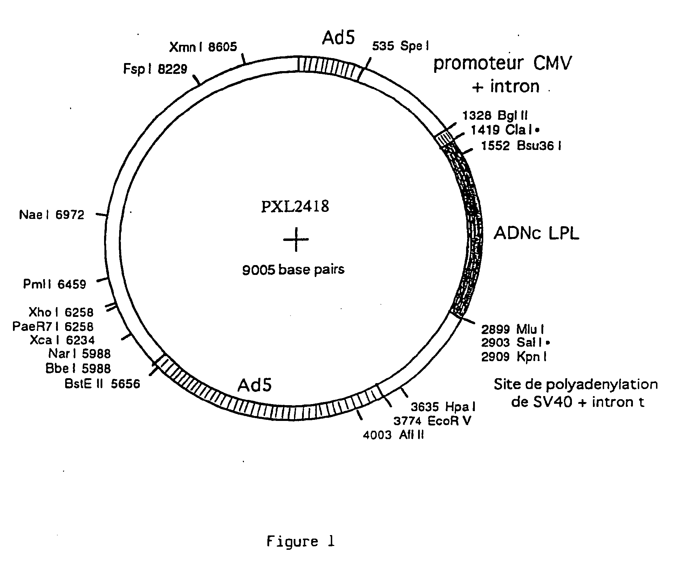

### Exemple 1. Construction du vecteur pXL2418 portant le gène codant pour la LPL sous le contrôle du promoteur précoce du cytomégalovirus (CMV) (figure 1).

Cet exemple décrit la construction d'un vecteur comprenant une séquence d'ADNc codant pour la LPL, sous le contrôle d'un promoteur constitué par le promoteur précoce du cytomégalovirus (CMV), ainsi qu'un région du génome de l'adénovirus Ad5 permettant la recombinaison homologue. Ce vecteur a été construit comme décrit ci-après.
1.1. Construction du vecteur pXL2375 : Le vecteur pXL2375 contient notamment une région du génome de l'adénovirus Ad5 et une séquence d'ADN codant pour l'apolipoprotéine AI sous le controle du promoteur CMV. Plus particulièrement, le promoteur CMV utilisé s'étend jusqu'au site 5' donneur d'épissage lié aux 107 pb les plus en 3' de l'intron synthétique décrit par O'Gorman et al. (Science 251 (1991) 1351). La construction de ce vecteur a été décrite en détail dans la demande co-pendante FR 93 05125. Il est entendu que des constructions similaires peuvent être réalisées par l'homme du métier.
1.2. Construction d'une séquence d'ADNc codant pour la LPL.
   Le plasmide pHLPL 26-1 décrit par Wion et al. (Science 235 (1987) 1638-1641) contient une séquence incomplète de l'ADNc de la LPL. Ainsi, ce plasmide contient les bases 272 à 1623 de l'ADNc de la LPL bordées par deux sites EcoRI, clonées au site EcoRI d'un plasmide pGEM1 (Promega).
   Le fragment EcoRI de pHLPL 26-1 contenant l'ADNc partiel de la LPL a été recloné dans le site EcoRI d'un plasmide pMTL22 (Chambers et al, Gene, 1988, 68:138-149), dans l'orientation mettant les bases 5' de l'ADNc du coté du site BglII de pMTL22. Le plasmide résultant a été appelé pXL2402.
   Les ARN de tissus adipeux humain ont ensuite été extraits selon la technique de Chromczynski et Sacchin (Anal. Biochem. 162 (1987) 156-159). A partir de cette préparation d'ARN, une amplification a été réalisée par RT-PCR, afin d'isoler la partie manquante de l'ADNc de la LPL. Pour cela, les amorces suivantes ont été utilisées :
   Sq4541 : TTA GAT CTA TCG ATA GAT GGA GAG CAA AGC CCC TG (SEQ ID n°1)
   Cette amorce permet d'introduire un site BglII ainsi qu'un site ClaI en amont de l'ATG.
   Sq3810 : TAC ATT CCT GTT ACC GTC CAG CCA TGG ATC (SEQ ID n° 2)
   Le fragment PCR de 260pb obtenu après 25 cycles d'amplification a ensuite été cloné dans le plasmide pCR-II (Invitrogen) et séquencé pour vérification. Il a ensuite été introduit par les site BglII et NcoI dans le vecteur pXL2402, ce qui reconstitue un ADNc complet précédé d'un site ClaI et suivi d'un site SalI. Le plasmide résultant a été appelé pXL2417.
1.3. Construction du vecteur pXL2418.
   L' ADNc de LPL a enfin été inséré dans le plasmide pXL2375, entre les sites SalI et ClaI, suite à l'excision par ces deux mêmes enzymes de l'ADNc de l'Apo A1. Le plasmide obtenu a été désigné pXL2418 (figure 1).

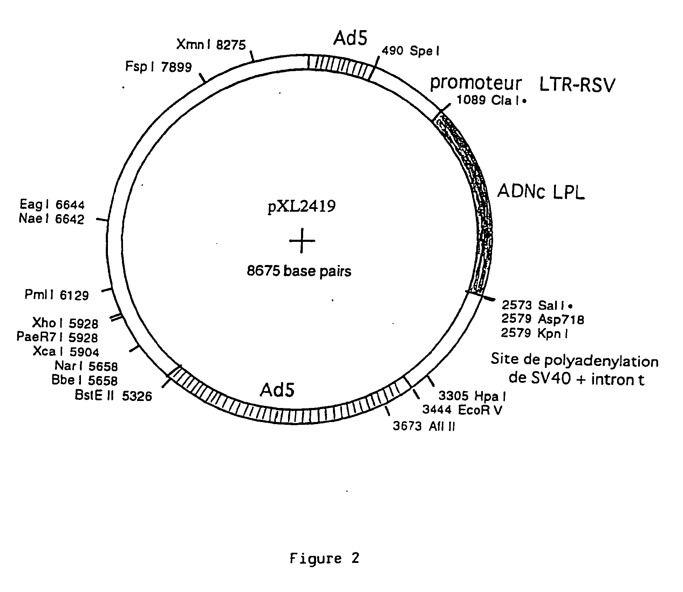

### Exemple 2. Construction du vecteur pXL2419 portant le gène codant pour la LPL sous le contrôle du promoteur du LTR du virus du sarcome de rous (LTR-RSV) (figure 2).

Cet exemple décrit la construction d'un vecteur comprenant une séquence d'ADNc codant pour la LPL, sous le contrôle d'un promoteur constitué par le LTR du virus du sarcome de rous (LTR-RSV), ainsi qu'un région du génome de l'adénovirus Ad5 permettant la recombinaison homologue. Ce vecteur a été construit comme décrit ci-après.
2.1. Construction du vecteur pXL2244 : Le vecteur pXL2244 contient notamment une région du génome de l'adénovirus Ad5 et une séquence d'ADN codant pour l'apolipoprotéine AI sous le controle du promoteur LTR-RSV.
2.2. Construction d'une séquence d'ADNc codant pour la LPL.
   La séquence d'ADNc codant pour la LPL utilisée dans cet exemple est celle décrite dans l'exemple 1.2.
2.3. Construction du vecteur pXL2419.
   L' ADNc de LPL a été inséré dans le plasmide pXL2244, entre les sites SalI et ClaI, suite à l'excision par ces deux mêmes enzymes de l'ADNc de l'Apo A1. Le plasmide obtenu a été désigné pXL2419 (figure 2).

### Exemple 3 : Construction des vecteurs pXL RSV-LPL et pXL CMV-LPL

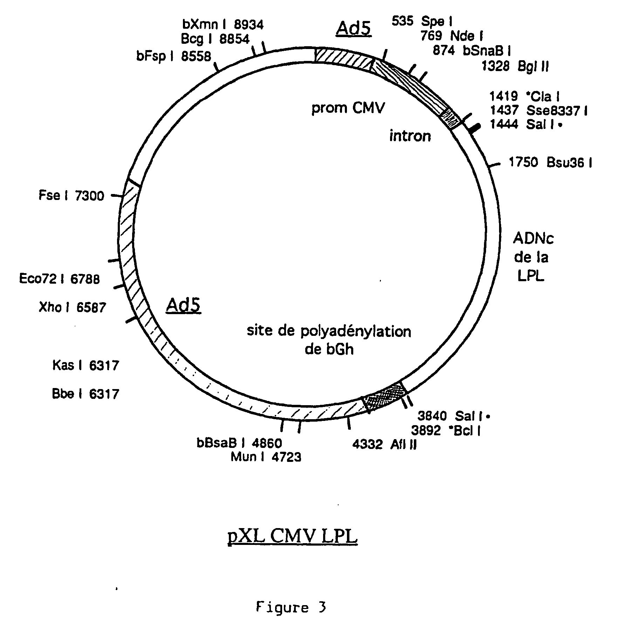

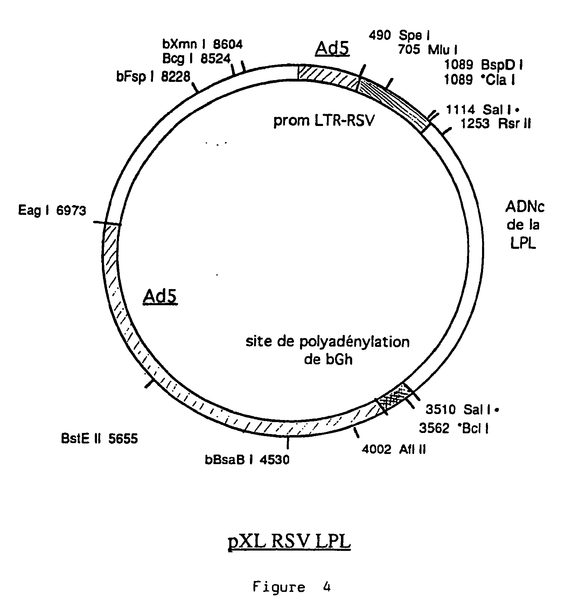

Le vecteur pRC-CMV LPL contient un fragment d'ADNc de la LPL s'étendant des bases 1 à 2388 de la séquence publiée par Wion et al, clonée aux sites HindIII et XbaI du vecteur d'expression pRC-CMV (Invitrogen). Le site HindIII a été modifié en un site ClaI par insertion de l'oligonucléotide AGC TAC ATC GAT GT (SEQ ID n° 3). L'ADNc de la LPL et le site de polyadénylation de l'hormone de croissance bovine (initialement contenu dans pRC-CMV) sont finalement extrait du pRCMV-LPL par coupure SphI, traitement à la T4 polymérase et coupure ClaI. Le fragment ainsi obtenu est cloné dans les vecteurs pXL2418 (exemple 1) et pXL2419 (exemple 2) coupés par ClaI et EcoRV, générant respectivement les vecteurs pXL CMV-LPL (figure 3) et pXL RSV-LPL (figure 4).

### Exemple 4 : Construction du vecteur pXL RSV-LPLc.

Cet exemple décrit la construction d'un vecteur utilisable pour générer des virus recombinants contenant un ADNc court codant pour la LPL.

Un ADNc plus court (bases 146 à 1635 de la séquence de Wion et al) a été cloné à partir des ARN de tissu adipeux humain. Les amorces ATC GGA TCC ATC GAT GCA GCT CCT CCA GAG GGA CGC (SEQ ID n° 4) et ATC TCT AGA GTC GAC ATG CCG TTC TTT GTT CTG TAG (SEQ ID n° 5), qui créent respectivement un site BamHI et un site ClaI en 5' de l'ADNc ainsi qu'un site XbaI et un site SalI en 3' de l'ADNc de la LPL ont été utilisées.

Ce fragment PCR est cloné dans PCR II et sa séquence intégralement vérifiée. L'ADNc de la LPL est ensuite resorti par les sites BamHI et XbaI et cloné dans un vecteur d'expression pcDNA3 (Invitrogen) pour vérification de l'expression, générant le plasmide pcDNA3-LPLc.

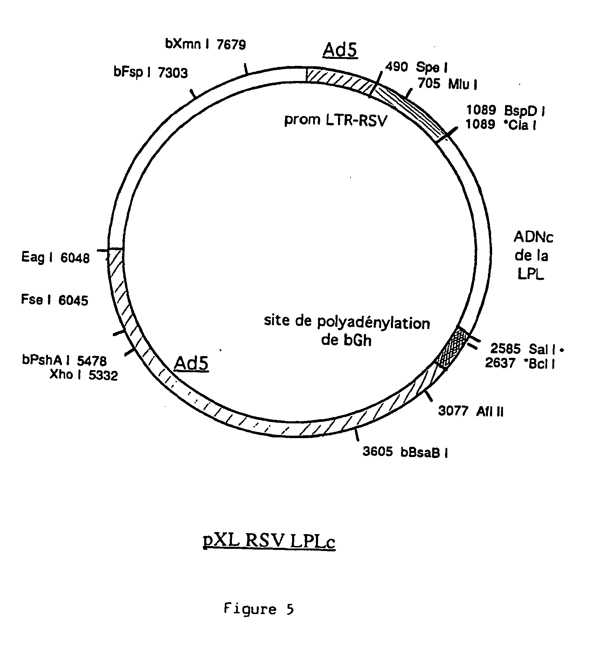

Le fragment ClaI -Sali contenant l'ADNc de la LPL est finalement cloné aux mêmes sites dans le plasmide pXL RSV-LPL (exemple 3) pour générer le plasmide navette pXL RSV-LPLc (figure 5).

### Exemple 5 : Fonctionnalité des vecteurs de l'invention : mise en évidence d'une activité LPL.

La capacité des vecteurs de l'invention à exprimer sur culture cellulaire une forme biologiquement active de la LPL a été démontrée par transfection transitoire de cellules 293 ou CosI. Pour cela, les cellules (2.10⁶ cellules par boite de 10 cm de diamètre) ont été transfectées (8 µg de vecteur) en présence de Transfectam. L'expression de la séquence codant pour la LPL et la production d'une protéine biologiquement active sont mises en évidence soit en masse, à l'aide d'un test immunoenzymatique (5.1.), soit en activité lipase (5.2.).
5.1. Mesure en masse de la LPL
   Une plaque ELISA Immulon I (Dynatech) est coatée par des anticorps monoclonaux anti-LPL bovine cross-réagissant avec la LPL humaine (20µg/ml en PBS, 50µl/puits). Les sites potentiels restant dans les puits sont ensuite bloqués (saturés) par incubation en présence de gélatine à 1%, 1 heure à température ambiante. Les échantillons à mesurer sont ensuite incubés 1 heure à 37°C.
   La révélation est faite par un sérum anti LPL-dilué à 10µg/ml, 100µl/puits, suivi d'un antisérum marqué à la peroxydase. L'activité peroxydase est détectée à l'aide d'un substrat TMB (kit Kirkegaard et Perry Laboratories inc.) et lecture de l'absorbance à 490nm.
5.2. mesure de l'activité LPL
   L'activité lipase totale est mesurée sur un substrat composé d'une émulsion de 0,3mg de trioléine (Sigma), 75nCi de tri(1-¹⁴C)oleoyl glycérol (55mCi/mmol, Amersham), 18mg de BSA (FractionV, Sigma), 25µl de plasma humain normal comme source d'ApoCII, le tout dans un volume final de 500µl de Tris 0,223M pH 8,5. De façon générale, l'activité est mesurée sur 100µl de surnageant de cellules transfectées ou 50µl de plasma post-héparine.
   Après 1 heure d'incubation à 37°C, la réaction est stoppée par l'addition de 3,25ml de tampon d'extraction (Chloroforme/méthanol/heptane, 10:9:7, v/v/v) et 0,75ml de tampon carbonate/borate pH 10,5 et la phase organique comptée pour déterminer la quantité d'acides gras libérés.
   Pour déterminer l'activité spécifiquement liée à la LPL , la mesure de l'activité hépatique lipase est faite en présence d'une concentration de 1M de NaCl (ce qui inhibe la LPL) puis soustraite de l'activité totale. Il est également possible d'inhiber l'activité lipoprotéine lipase par un anticorps monoclonal spécifique (Babirak et al, Atheriosclerosis, 1989, 9:326-334).

Les plasmides pXL RSV-LPL et pXL CMV-LPL ont été testés par transfection dans des cellules CosI par comparaison avec le plasmide pRC CMV-LPL. Les résultats sont présentés dans le tableau 1.

**Tableau 1**

| Vecteur d'expression | Activité dans le surnagent Jour 1 |
|---|---|
| pRC-CMV-LPL | 24,5 |
| pXL RSV-LPL | 15,1 |
| pXL CMV-LPL | 22,9 |

Le plasmide pcDNA-LPLc a été testé par transfection dans des cellules 293 par comparaison avec un vecteur d'expresssion pcDNA3 contenant le même ADNc que le vecteur pRC-CMV-LPL. Les résultat sont présentés dans le tableau 2.

**Tableau 2**

| Vecteur d'expression | Activité dans le surnagent Jour 1 | Activité dans le surnagent Jour 2 |
|---|---|---|
| pcDNA3-LPL | 106,4 mU/ml | 106,7 mU/ml |
| pcDNA3-LPLc | 114 mU/ml | 109,6 mU/ml |

### Exemple 6. Construction d'un adénovirus recombinant Ad-CMV.LPL contenant une séquence codant pour la lipase LPL

Les plasmides préparés dans les exemples 1 à 4 sont linéarisés et cotransfectés pour la recombinaison avec le vecteur adénoviral déficient, dans les cellules helper (lignée 293) apportant en *trans* les fonctions codées par les régions E1 (E1A et E11B) d'adénovirus.

Plus particulièrement, l'adénovirus Ad.CMV.LPL est obtenu par recombinaison homologue in vivo entre l'adénovirus Ad.RSVßgal (Stratford-Perricaudet et al., J. Clin. Invest 90 (1992) 626) et le plasmide pXL2418 ou pXL CMV-LPL selon le protocole suivant : Le plasmide pXL2418 ou pXL CMV-LPL linéarisé et l'adénovirus Ad.RSVßgal linéarisé par ClaI sont co-transfectés dans la lignée 293 en présence de phosphate de calcium pour permettre la recombinaison homologue. Les adénovirus recombinants ainsi générés sont sélectionnés par purification sur plaque. Après isolement, l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.

Les particules virales sont ensuite purifiées par centrifugation sur gradient de chlorure de césium selon les techniques connues (voir notamment Graham et al., Virology 52 (1973) 456). L'adénovirus Ad-CMV.LPL peut être conservé à -80°C dans 20 % de glycérol.

Le même protocole est reproduit avec le plasmide pXL2419 ou pXL RSV-LPL ou pXL RSV-LPLc, conduisant à l'adénovirus recombinant Ad.RSV.LPL ou Ad.RSV.LPLc.

### Exemple 7 : Transfert in vivo du gène LPL par un adénovirus recombinant

Cet exemple décrit le transfert du gène LPL in vivo au moyen d'un vecteur adénoviral selon l'invention.

Les adénovirus injectés sont les adénovirus Ad-CMV.LPL et Ad.LTR.LPL préparés dans l'exemple 5, utilisés sous forme purifiée (3,5 10⁶ pfu/µl), dans une solution saline phosphate (PBS). Ces virus sont injectés dans des souris C57B1/6 par voie intra veineuse en utilisant la veine de la queue, le sinus rétro-orbital ou la veine porte. L'expression d'une forme active de LPL est mise en évidence dans les conditions décrites dans l'exemple 5.

## Revendications

1. Virus recombinant défectif à l'exclusion du phagémide CDM8 comprenant une séquence nucléique codant pour pour tout ou partie de la lipoprotéine lipase (LPL) ou d'un dérivé de celle-ci

2. Virus selon la revendication 1 **caractérisé en ce que** la séquence d'ADN est une séquence d'ADNc.

3. Virus selon la revendication 1 **caractérisé en ce que** la séquence d'ADN est une séquence d'ADNg.

4. Virus selon l'une des revendications 1 à 3 **caractérisé en ce que** la séquence d'ADN code pour la LPL humaine.

5. Virus selon l'une des revendications 1 à 4 **caractérisé en ce que** la séquence d'ADN est placée sous le contrôle de signaux permettant son expression dans les cellules infectées.

6. Virus selon la revendication 5 **caractérisé en ce que** les signaux d'expression sont choisis parmi les promoteurs viraux.

7. Virus selon la revendication 6 **caractérisé en ce que** les signaux d'expression sont choisis parmi les promoteurs E1A, MLP, CMV et LTR-RSV.

8. Virus recombinant défectif comprenant une séquence d'ADNc codant pour la lipoprotéine lipase sous le contrôle d'un promoteur choisi parmi le promoteur LTR-RSV et le promoteur précoce du CMV.

9. Virus selon l'une des revendications précédentes **caractérisé en ce qu'**il comprend une séquence permettant d'orienter la lipoprotéine lipase dans les voies de sécrétion de la cellule infectée.

10. Virus selon la revendication 9 **caractérisé en ce que** la séquence de sécrétion est la séquence native de la lipoprotéine lipase.

11. Virus selon l'une des revendications 1 à 10 **caractérisé en ce qu'**il est dépourvu des régions de son génome qui sont nécessaires à sa réplication dans la cellule cible.

12. Virus selon l'une des revendications 1 à 11 **caractérisé en ce qu'**il est dépourvu des régions de son génome qui sont nécessaires à sa réplication dans la cellule cible.

13. Virus selon l'une des revendications 1 à 12 **caractérisé en ce qu'**il s'agit d'un adénovirus.

14. Virus selon la revendication 13 **caractérisé en ce qu'**il sagit d'un adénovirus humain de type Ad 2 ou Ad 5 ou canin de type CAV-2.

15. Virus selon l'une des revendications 1 à 12 **caractérisé en ce qu'**il s'agit d'un virus adéno-associé.

16. Virus selon l'une des revendications 1 à 12 **caractérisé en ce qu'**il s'agit d'un rétrovirus.

17. Virus selon l'une des revendications 1 à 12 **caractérisé en ce qu'**il s'agit d'un virus de l'herpès (HSV).

18. Utilisation d'un virus selon l'une des revendications 1 à 17 pour la préparation d'une composition pharmaceutique destinée au traitement et/ou à la prévention des pathologies liées aux dyslipoprotéinémies.

19. Composition pharmaceutique comprenant un ou plusieurs virus recombinants défectifs selon l'une des revendications 1 à 17.

20. Composition pharmaceutique selon la revendication 19 **caractérisée en ce qu'**elle est sous forme injectable.

21. Composition pharmaceutique selon la revendication 19 ou 20 **caractérisée en ce qu'**elle comprend entre 10⁴ et 10¹⁴ pfu/ml, et de préférence 10⁶ à 10¹⁰ pfu/ml adénovirus recombinants défectifs.

22. Cellule isolée de mammifère infectée par un ou plusieurs virus recombinants défectifs selon l'une des revendications 1 à 17.

23. Cellule selon la revendication 22 **caractérisée en ce qu'**il s'agit d'une cellule humaine.

24. Implant comprenant des cellules selon la revendication 23 **caractérisé en ce que** la matrice extracellulaire comprend un composé gélifiant choisi de préférence parmi le collagène, la gélatine, les glycosaminoglycans, la fibronectine et les lectines.

25. Implant comprenant des cellules selon la revendication 23 ou implant selon la revendication 24 **caractérisé en ce que** la matrice extracellulaire comprend également un support permettant l'ancrage des cellules infectées.

26. Implant selon la revendication 25 **caractérisé en ce que** le support est constitué préférentiellement par des fibres de polytétrafluoroéthylène.

27. Adénovirus recombinant défectif selon la revendication 13 comportant en plus un gène codant pour une apolipoprotéine.

## Claims

1. Defective recombinant virus, excluding phagemid CDM8, comprising a nucleic acid sequence encoding all or part of lipoprotein lipase (LPL) or of a derivative thereof.

2. Virus according to Claim 1, **characterized in that** the DNA sequence is a cDNA sequence.

3. Virus according to Claim 1, **characterized in that** the DNA sequence is a gDNA sequence.

4. Virus according to one of Claims 1 to 3, **characterized in that** the DNA sequence encodes human LPL.

5. Virus according to one of Claims 1 to 4, **characterized in that** the DNA sequence is placed under the control of signals which allow its expression in the infected cells.

6. Virus according to Claim 5, **characterized in that** the expression signals are chosen from viral promoters.

7. Virus according to Claim 6, **characterized in that** the expression signals are chosen from the E1A, MLP, CMV and LTR-RSV promoters.

8. Defective recombinant virus comprising a cDNA sequence encoding lipoprotein lipase under the control of a promoter chosen from the LTR-RSV promoter and the CMV early promoter.

9. Virus according to one of the preceding claims, **characterized in that** it comprises a sequence which makes it possible to orient the lipoprotein lipase into the secretion pathways of the infected cell.

10. Virus according to Claim 9, **characterized in that** the secretion sequence is the native sequence of the lipoprotein lipase.

11. Virus according to one of Claims 1 to 10, **characterized in that** it lacks the regions of its genome which are necessary for its replication in the target cell.

12. Virus according to one of Claims 1 to 11, **characterized in that** it lacks the regions of its genome which are necessary for its replication in the target cell.

13. Virus according to one of Claims 1 to 12, **characterized in that** it is an adenovirus.

14. Virus according to Claim 13, **characterized in that** it is a human adenovirus type Ad2 or Ad5 or a canine adenovirus type CAV-2.

15. Virus according to one of Claims 1 to 12, **characterized in that** it is an adeno-associated virus.

16. Virus according to one of Claims 1 to 12, **characterized in that** it is a retrovirus.

17. Virus according to one of Claims 1 to 12, **characterized in that** it is a herpesvirus (HSV).

18. Use of a virus according to one of Claims 1 to 17, for preparing a pharmaceutical composition intended for the treatment and/or the prevention of dyslipoproteinaemia-related pathologies.

19. Pharmaceutical composition comprising one or more defective recombinant viruses according to one of Claims 1 to 17.

20. Pharmaceutical composition according to Claim 19, **characterized in that** it is in injectable form.

21. Pharmaceutical composition according to Claim 19 or 20, **characterized in that** it comprises between 10⁴ and 10¹⁴ pfu/ml, and preferably 10⁶ to 10¹⁰ pfu/ml, of defective recombinant adenoviruses.

22. Isolated mammalian cell infected with one or more defective recombinant viruses according to one of Claims 1 to 17.

23. Cell according to Claim 22, **characterized in that** it is a human cell.

24. Implant comprising cells according to Claim 23, **characterized in that** the extracellular matrix comprises a gelling compound preferably chosen from collagen, gelatine, glycosaminoglycans, fibronectin and lectins.

25. Implant comprising cells according to Claim 23 or implant according to Claim 24, **characterized in that** the extracellular matrix also comprises a support for anchoring the infected cells.

26. Implant according to Claim 25, **characterized in that** the support preferentially consists of polytetrafluoroethylene fibres.

27. Defective recombinant adenovirus according to Claim 13, also comprising a gene encoding an apolipoprotein.

## Patentansprüche

1. Rekombinantes defektes Virus, mit Ausnahme des Phagemids CDM8, welches eine Nukleinsäuresequenz umfasst, die für einen Teil oder die Gesamtheit der Lipoproteinlipase (LPL) oder eines Derivats derselben codiert.

2. Virus, **dadurch gekennzeichnet, dass** die DNA-Sequenz eine cDNA-Sequenz ist.

3. Virus nach Anspruch 1, **dadurch gekennzeichnet, dass** die DNA-Sequenz eine gDNA-Sequenz ist.

4. Virus nach einem der Ansprüche 1 und 2, **dadurch gekennzeichnet, dass** die DNA-Sequenz für menschliche LPL kodiert.

5. Virus nach einem der Ansprüche 1 bis 3, **dadurch gekennzeichnet, dass** die DNA-Sequenz der Steuerung durch Signale unterstellt ist, die ihre Expression in den infizierten Zellen ermöglichen.

6. Virus nach Anspruch 4, **dadurch gekennzeichnet, dass** die Expressionssignale aus viralen Promotoren ausgewählt sind.

7. Virus nach Anspruch 5, **dadurch gekennzeichnet, dass** die Expressionssignale aus den Promotoren E1A, MLP, CMV und LTR-RSV ausgewählt sind.

8. Defektes rekombinantes Virus, umfassend eine für Lipoproteinlipase kodierende cDNA-Sequenz, die von einem Promotor gesteuert ist, der aus dem Promotor LTR-RSV und dem frühen CMV-Promotor ausgewählt ist.

9. Virus nach einem der vorangegangenen Ansprüche, **dadurch gekennzeichnet, dass** es eine Sequenz umfasst, die das Hinleiten der Lipoproteinlipase in die Sekretionswege der infizierten Zelle ermöglicht.

10. Virus nach Anspruch 8, **dadurch gekennzeichnet, dass** die Sekretionssequenz die native Sequenz der Lipoproteinlipase ist.

11. Virus nach einem der Ansprüche 1 bis 9, **dadurch gekennzeichnet, dass** sein Genom keine Regionen umfasst, die für seine Replikation in der Zielzelle notwendig sind.

12. Virus nach einem der Ansprüche 1 bis 10, **dadurch gekennzeichnet, dass** sein Genom keine Regionen umfasst, die für seine Replikation in der Zielzelle notwendig sind.

13. Virus nach einem der Ansprüche 1 bis 11, **dadurch gekennzeichnet, dass** es sich um ein Adenovirus handelt.

14. Virus nach Anspruch 12, **dadurch gekennzeichnet, dass** es sich um ein humanes Adenovirus Typ Ad2 oder Ad5 oder um ein canines Adenovirus vom Typ CAV-2 handelt.

15. Virus nach einem der Ansprüche 1 bis 11, **dadurch gekennzeichnet, dass** es sich um ein adenoassoziiertes Virus handelt.

16. Virus nach einem der Ansprüche 1 bis 11, **dadurch gekennzeichnet, dass** es sich um ein Retrovirus handelt.

17. Virus nach einem der Ansprüche 1 bis 11, **dadurch gekennzeichnet, dass** es sich um ein Herpesvirus (HSV) handelt.

18. Verwendung eines Virus nach einem der Ansprüche 1 bis 16 zur Herstellung einer pharmazeutischen Zusammensetzung zum Zweck der Behandlung und/oder der Vorbeugung von Krankheiten, die mit Dyslipoproteinämien in Zusammenhang stehen.

19. Pharmazeutische Zusammensetzung, umfassend ein oder mehrere defekte rekombinante Viren nach einem der Ansprüche 1 bis 16.

20. Pharmazeutische Zusammensetzung nach Anspruch 18, **dadurch gekennzeichnet, dass** diese in injizierbarer Form vorliegt.

21. Pharmazeutische Zusammensetzung nach Anspruch 18 oder 19, **dadurch gekennzeichnet, dass** diese zwischen 10⁴ und 10¹⁴ pfu/ml, vorzugsweise 10⁸ bis 10¹⁰ pfu/ml defekte rekombinante Adenoviren umfasst.

22. Isolierte Zelle eines Säugetiers, die mit einem oder mehreren defekten rekombinanten Viren nach einem der Ansprüche 1 bis 16 infiziert ist.

23. Zelle nach Anspruch 21, **dadurch gekennzeichnet, dass** es sich um eine menschliche Zelle handelt.

24. Implantat, umfassend Zellen nach Anspruch 22, **dadurch gekennzeichnet, dass** die extrazelluläre Matrix eine Gelierverbindung umfasst, die vorzugsweise aus Collagen, Gelatine, Glykosaminglykanen, Fibronektin und Lektinen ausgewählt ist.

25. Implantat, umfassend Zellen nach Anspruch 22, oder Implantat nach Anspruch 23, **dadurch gekennzeichnet, dass** die extrazelluläre Matrix zudem einen Träger umfasst, der die Verankerung der infizierten Zellen ermöglicht.

26. Implantat nach Anspruch 24, **dadurch gekennzeichnet, dass** der Träger vorzugsweise aus Polytetrafluorethylenfasern besteht.

27. Defektes rekombinantes Adenovirus nach Anspruch 12, ferner umfassend ein Gen, das für ein Apolipoprotein kodiert.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: : VIRUS RECOMBINANTS, PREPARATION ET UTILISATION EN THERAPIE GENIQUE.
   (iii) NOMBRE DE SEQUENCES: 5
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEO ID NO: 1:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE :
      (D) AUTRE INFORMATION: /product= Sq4541
   (xi) DESCRIPTION DE LA SEQUENCE: SEQ ID NO: 1:
      TTAGATCTAT CGATAGATGG AGAGCAAAGC CCCTG 35
(2) INFORMATION POUR LA SEO ID NO: 2:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (ix) CARACTERISTIQUE :
      (D) AUTRE INFORMATION: /product= Sq3810
   (xi) DESCRIPTION DE LA SEQUENCE: SEQ ID NO: 2:
      TACATTCCTG TTACCGTCCA GCCATGGATC 30
(2) INFORMATION POUR LA SEO ID NO: 3:
   (i) CARACTERISTIQUES DE LA SEQUENCE:
      (A) LONGUEUR: 14 paires de bases
      (B) TYPE: acide nucléique
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 3:
      AGCTACATCG ATGT 14
(2) INFORMATION POUR LA SEO ID NO: 4:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
      ATCGGATCCA TCGATGCAGC TCCTCCAGAG GGACGC 36
(2) INFORMATION POUR LA SEO ID NO: 5:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
      ATCTCTAGAG TCGACATGCC GTTCTTTGTT CTGTAG 36